Europäisches Patentamt

European Patent Office

Office européen des brevets

(19)

(11) Publication number: **0 176 621**
**B1**

(12) **EUROPEAN PATENT SPECIFICATION**

(45) Date of publication of patent specification: **23.03.88**

(21) Application number: **84111941.5**

(22) Date of filing: **05.10.84**

(51) Int. Cl.⁴: **C 12 P 19/20, C 12 P 19/22,
C 13 K 1/06, C 13 K 7/00**

(54) Glucose or maltose from starch.

(43) Date of publication of application:
**09.04.86 Bulletin 86/15**

(45) Publication of the grant of the patent:
**23.03.88 Bulletin 88/12**

(84) Designated Contracting States:
**BE DE FR GB IT NL**

(56) References cited:
**EP-A-0 136 087
GB-A-2 045 764
US-A-3 668 007
US-A-3 720 583**

(73) Proprietor: **UOP Inc.
10 UOP Plaza Algonquin & Mt. Prospect Roads
Des Plaines Illinois 60016 (US)**

(72) Inventor: **Rohrbach, Ronald Paul
21 W. 755 Ravine Road
Forest Lake Illinois 60047 (US)**
Inventor: **Maliarik, Mary Jon
1103 Maiden Lane Court, No. 106
Ann Arbor Michigan 48105 (US)**
Inventor: **Malloy, Thomas Patrick
730 Red Bridge
Lake Zurich Illinois 60007 (US)**
Inventor: **Thompson, Gregory John
419 Lorraine Avenue
Waukegan Illinois 60085 (US)**
Inventor: **Lin, Kaung Far
730 South Beverley Lane
Arlington Heights Illinois 60005 (US)**
Inventor: **Penner, David Wayne
1595 Buckthorn
Hoffman Estates Illinois (US)**

(74) Representative: **Weber, Dieter, Dr. et al
Dr. Dieter Weber und Klaus Seiffert
Patentanwälte Gustav-Freytag-Strasse 25
Postfach 6145
D-6200 Wiesbaden 1 (DE)**

# 0 176 621

**Description**

## BACKGROUND OF THE INVENTION

Naturally occurring carbohydrates in the form of starch are renewable sources of commercially important sugars including the monosaccharide glucose and the disaccharide maltose. Glucose and maltose are useful food sweeteners and nutrients. Maltose is even useful in culture media. Glucose is especially useful because it can be isomerized to form the even sweeter monosaccharide, fructose. This invention relates to a two step process for obtaining high purity glucose or maltose from a feedstock comprising starch derived from such plants as cassava, maize, potatoes, rice, tapioca, taro and wheat.

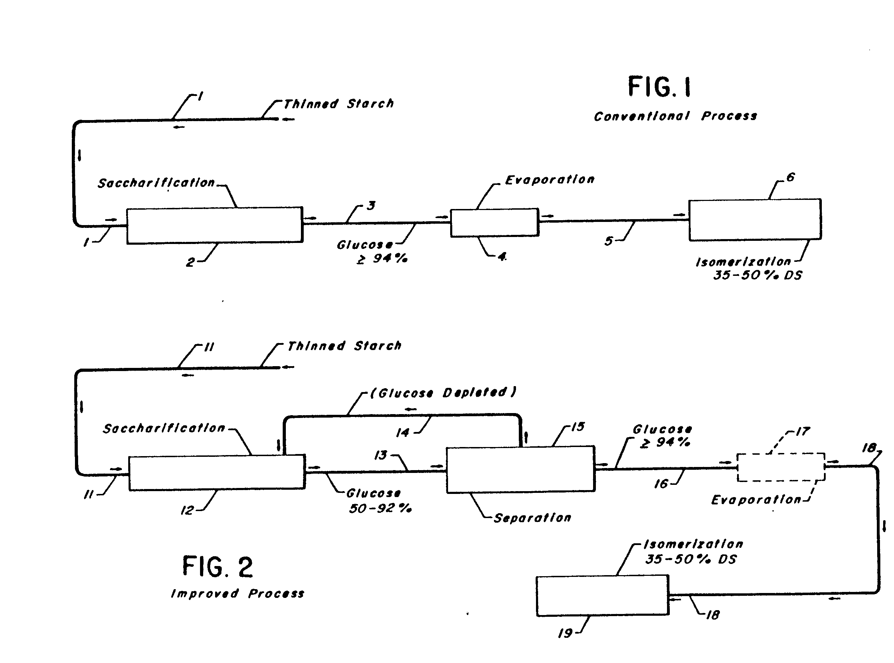

The conventional scheme for glucose production from thinned starch is depicted in Figure 1. In Figure 1, and throughout the specification, the term "thinned starch" refers to a liquified starch partially hydrolyzed by an alpha-amylase enzyme. Different types of alpha-amylase enzymes are often used to produce thinned starch with different, but analogous properties depending upon whether maltose or glucose is desired. Thinned starch has a dry solids (DS) level of 30—45% and contains a minor proportion of monosaccharides, up to 10% but usually less than 4%, 20% to 70% disaccharides through heptasaccharides ($DP_2$—$DP_7$), and 30% to 80% octasaccharides and the higher molecular weight polysaccharides. One measure of its degree of hydrolysis is the dextrose equivalent. A thinned starch is said to have an increased dextrose equivalent, that is, an increased proportion of dextrorotatory glucose when compared to untreated starch. Untreated starch contains no or few free units of dextrose; thinned starch may have the dextrose concentration increased to a low value of from 5 to 25.

In the conventional process of Figure 1, thinned starch *1* enters a saccharification or hydrolysis reactor zone, *2*, where it undergoes enzyme-catalyzed hydrolysis using a glucose-forming enzyme, for example, amylglucosidase (glucoamylase), hereafter referred to as AG. An essential feature of conventional processes is that hydrolysis is continued in one step until maximum glucose formation is attained, which corresponds to about 94—96% glucose in the product stream, *3*, when using a feedstock containing about 30% dry solids. Although only one reactor zone is depicted for saccharification, this is but one embodiment, and a plurality of reactor zones in series may be used in other embodiments.

Effluent, *3*, from *2* containing more than about 94% glucose (on a dry solids basis) is then concentrated, where necessary, in an evaporation zone *4*, to afford a product stream, *5*, containing from about 35% to about 50% dry solids. This product stream, *5*, is typically the feedstock entering an isomerization reactor zone, *6*, in which glucose is enzymatically converted to fructose by glucose isomerase.

The conventional preparation of maltose from starch is analogous to that depicted in Figure 1, the major difference being that the thinned starch is hydrolyzed with a maltose-producing enzyme. Beta-amylase is largely or exclusively the maltose-producing enzyme used.

Our invention is directed toward both glucose and maltose production. However, solely for the sake of brevity this description following will be directed toward glucose production, with it being clearly understood that a similar description directed toward maltose production is contemplated.

Several disadvantages attach to the conventional process. One disadvantage, generic to any run to maximum conversion, is the increased cost consequent to the process time requirements for attaining maximum conversion: the longer the time for such conversion to be established, the more costly, hence more disadvantageous, is the process. Because glucose represses enzyme activity by complexing with AG, the hydrolysis rate is decreased as glucose accumulates and further increases process time. Another disadvantage characteristic of the relatively long residence time associated with the AG-catalyzed hydrolysis of thinned starch, is that the glucose, a monosaccharide, reverts to disaccharides, among which is iso-maltose. Because isomaltose is a refractory disaccharide, that is, it is not readily hydrolyzed, and because it is bitter, it is a highly undesirable component of a glucose feedstock used for fructose production. The longer the reaction time, the higher the glucose level and a higher isomaltose concentration in the product results.

A still further disadvantage, at least where a soluble enzyme is used in the saccharification zone, is that the enzyme must be continuously replaced as it is lost during production. Cost rises directly as the amount of glucose formed per unit of enzyme decreases.

The US—A—3 720 583 describes a process for producing a sugar from thinned starch by hydrolyzing the feedstock under the action of an enzyme, separating the effluent by ultrafiltration into a sugar-enriched product stream and a sugar-depleted stream and recycling the sugar-depleted stream to the hydrolysis step.

Because present commercial processes for production of high fructose corn syrup by isomerization of glucose utilize a glucose feedstock containing at least 94% glucose, a constraint of any new or modified process for production of glucose is that it affords comparable glucose levels.

Thus, the objective of the invention was to provide a product stream having at least 90% glucose or maltose and avoiding the aforementioned disadvantages of conventional processes.

The process according to the invention for selectively obtaining a sugar which is either glucose or maltose from thinned starch comprising hydrolyzing a feedstock of thinned starch under the action of a glucose- or maltose-producing enzyme, separating the effluent stream into a sugar-enriched product stream and a sugar-depleted stream, recovering the sugar-enriched product stream, and recycling the sugar-depleted stream to the hydrolysis step, is characterized in that the feedstock is hydrolyzed to an effluent stream containing from 50% to 92% of said sugar.

By carrying out hydrolysis to a state substantially short of maximum glucose formation, the invention herein achieves a considerable saving time and affords glucose with substantially lower levels of reversion products.

Therefore an advantage of our invention is that it affords a substantial reduction in process time. Another advantage accompanying a reduction in time is that the process of our invention affords glucose with less reversion products than the prior art processes.

Still other advantages accrue from the characteristics of enzyme-catalyzed hydrolysis of thinned starch of which presently used commercial processes cannot take advantage. When a feedstock for AG-catalyzed hydrolysis is increased in dry solids, it is found that enzyme stability, as measured by its half-life, also increases. It is also found that the rate of glucose formation increases with increasing dry solids. Both of these characteristics are quite favorable, yet cannot be used in present commercial processes because increasing dry solids also leads to a lower maximum glucose level accompanied by increased reversion products.

In contrast to the prior art methods, the process of the instant invention is able to advantageously utilize the favorable characteristics of increased enzyme stability and increased glucose formation rate without any accompanying disadvantage of increased reversion products. Thus, in this sense our invention is truly synergistic; it incorporates the beneficial effects without incorporating the detrimental ones.

One characteristic of using immobilized AG in hydrolyzing thinned starch is that it typically affords less than 94% glucose at equilibrium and the heretofore relatively long residence time has resulted in the appearance of unwanted reversion products. Thus, immobilized AG can be used only with difficulty in present commercial processes. Therefore, yet another advantage of the instant invention is that it readily permits the use of an immobilized AG. One characteristic of using soluble (or unimmobilized) AG in hydrolyzing thinned starch is that it is necessary to continuously replace the enzyme which is lost during the production of glucose. Therefore, still another advantage of the process described herein is that it affords a substantial increase in productivity, defined as the amount of glucose formed per unit of enzyme. This productivity increase results, in part, from recycling the enzyme incidental to the recycle stage of the process (where soluble AG is used), as well as a longer half-life (where either a soluble or immobilized AG is used).

The glucose level in our process product stream as described is at least 90%. However, glucose levels of greater than 99% may be readily achieved by suitably varying process variables. Thus, still another advantage of our process is that it may be tailored to continually produce high-purity glucose, with a glucose purity greater than 99% being attainable.

Yet another advantage of the process which is our invention is that it can afford virtually complete reversion of starch to glucose.

It should be readily apparent from the multitude of the aforementioned advantages that our invention represents a substantial advance in the art of producing glucose at levels of about 94% and greater by enzyme-catalyzed hydrolysis of thinned starch.

In a more specific embodiment, the effluent from the hydrolysis step contains 70% to 80% sugar. In a still more specific embodiment, the sugar-enriched product stream contains at least 90% sugar.

## DESCRIPTION OF THE INVENTION

This invention is a process for obtaining glucose or maltose from thinned starch which represents a radical departure from prior art methods. One point of departure is the partial hydrolysis of thinned starch. That is to say, whereas the prior art methods hydrolyzed thinned starch for a time sufficient to attain maximum formation of glucose, the process herein continues hydrolysis for a substantially lesser period of time, thereby affording an effluent which contains less than maximum levels of glucose. The process herein is conveniently summarized by the flow diagram depicted in Figure 2.

In Figure 2, a thinned starch, 11, as defined within, usually containing from about 30% to about 45% dry solids, is the feedstock for a saccharification reactor zone, 12. Although only one reactor zone is depicted, this is but one embodiment. Embodiments where a plurality of reactor zones are used in the saccharification step are contemplated and are to be considered within the scope of the claimed invention. Effluent stream 13, from the saccharification zone contains glucose at levels based on total solids from about 50% to about 92%, and this is used as the feedstock for the separation step, 15. Separation is here depicted as a single stage, but embodiments employing multistage separation are variants within the scope of this invention.

The effluent from the separation zone is in two streams, one being product enriched in glucose, 16, to contain at least 90% glucose (on a total solids basis), the other being a glucose-depleted stream, 14. This latter is then recycled to the saccharification reactor zone, 12.

Where the glucose-enriched product stream 16 is ultimately to be used as the feedstock for a glucose isomerase reactor, it is then sent to an evaporation zone, 17, where necessary to afford stream 18 containing from about 35% to about 50% dry solids. Said stream 18 then is utilized as the feedstock for an isomerization reactor zone 19 converting glucose to fructose. However, it is to be clearly understood that the glucose-enriched product stream may serve as the glucose source for purposes other than use as a feedstock for isomerization to fructose. Other purposes to which the glucose may be put include hydrogenation to sorbitol, fermentation to ethanol, and a sweetener in food.

Where the product is maltose there is no isomerization reactor zone *19* as depicted in Figure 2. The maltose-enriched product stream corresponding to *16* is either used as is or sent to an evaporation zone *17* to afford more concentrated solutions of maltose, or even dry maltose.

In the initial step of our process, a feedstock of thinned starch is selectively enzymatically hydrolyzed by a glucose-producing enzyme, chiefly AG, to an effluent containing from about 50% to about 92% glucose. This selective hydrolysis step often is referred to as a saccharification step. The AG used may be soluble, in which case it is recycled with the glucose-depleted stream, or it may be an immobilized AG. In either case pullulanase or alpha-amylase, or both, may be present in the thinned starch feedstock to aid hydrolysis. The temperature at which the enzymatic hydrolysis is conducted depends upon the thermal stability of the enzyme used, but generally the temperature is between about 40° and 80°C, with the temperature of about 60°C being the most usual one. However, the AG from at least one microorganism is known to be sufficiently thermostable to allow the process to be run at temperatures even up to about 100°C. The pressure at which enzymatic hydrolysis is conducted is from 1 to 1000 pounds per square inch ($6.89 \times 10^3$ Pa to $6.89 \times 10^6$ Pa). The residence time during which the starch is in contact with immobilized enzyme is relatively short, that is, in a range of from 4 minutes to 1.6 hours and is in correlation with liquid hourly space velocities which may range from about 2 to about 50.

The space velocity and residence time are correlated in such a manner, i.e., short residence time and high space velocity within the ranges hereinbefore set forth so as to provide a conversion rate within the desired range. Conversely, it is also contemplated that long residence times and low space velocities with the aforesaid range may also be employed to effect the desired result.

The residence time during which the starch is in contact with soluble enzyme is relatively short in comparison to conventional processes, that is, in a range of from 2 hours to 48 hours.

A desirable consequence of hydrolyzing thinned starch to an effluent containing from 50% to 92% glucose is a substantial reduction in reaction time. Thus, hydrolysis to a product containing 50% glucose may take less than one-fourth of the time needed to attain 94% glucose, hydrolysis to 90% glucose may take only one-half the time, and even hydrolysis to 92% glucose may take only three-fifths the time. Another desirable consequence is a decided improvement in organoleptic characteristics through substantial reduction of the bitter principal, isomaltose; hydrolysis to 50% glucose may be accompanied by only about one-fourth as much isomaltose as accompanies the 94% glucose product.

A feedstock of thinned starch containing from about 30% to about 45% dry solids is conventional in the industry, although our process is not limited thereto. However, a dry solids level from about 35% to about 45% is preferred in the practice of this invention to obtain the full advantage of the salutary effect of higher dry solids on enzyme stability and the rate of glucose formation.

Prior art methods continue the hydrolysis to maximum glucose formation, which corresponds to glucose levels of about 94%. However, an essential feature of our process is continuation of hydrolysis to an effluent containing from 50% to 92%, but usually not more than 90% glucose. An effluent containing from about 60% to about 85% glucose is desirable, and one containing from about 70% to about 80% glucose is particularly preferred.

The hydrolysis effluent recovered from this saccharification step is then separated into a glucose-enriched product stream and a glucose-depleted stream in a separation step. Where the glucose-enriched product stream is used as the feedstock for isomerization to fructose, the stream will contain at least about 94% glucose since present processes for forming fructose from glucose require a feedstock containing at least about that level of glucose purity.

Where a feedstock of less than 94% glucose is acceptable, a less stringent separation to afford lower purity glucose may be effected. As a practical matter, the glucose-enriched product stream generally will contain at least about 90% glucose. It also must be understood that separation may be performed to obtain higher purity glucose, i.e., 94+% glucose. In fact, our invention may be used to continually produce glucose of greater than 99% purity where such high purity material is desired.

Generally, this separation step will comprise a single stage. However, multi-stage separation may be advantageous in some circumstances, and these are considered to be within the scope of our invention. In any event, the glucose-enriched product stream from the separation step is recovered for subsequent use or processing.

Any method of separation which is selective for glucose relative to disaccharides and higher polysaccharides is suitable. Where maltose, a disaccharide, is formed the separation needs to be selective relative only to higher polysaccharides. For example, a membrane-based separation may be effectively utilized. As another example, a separation based on solid adsorbents may be utilized. Examples of the latter include aluminas, silicas, various clays, zeolites, and so forth. Still another method of separation is selective crystallization of glucose from the saccharide mixture. Still other methods of separation which may be used in the process herein include solvent extraction and supercritical extraction, to cite but two further exemplary methods.

An integral part of this invention is the recycling of the glucose-depleted stream to the hydrolysis step. When a plurality of hydrolysis zones connected in series flow are used in the hydrolysis or saccharification step, the particular hydrolysis zone which is the entry point for the recycled stream will depend on process parameters such as reactor configuration, the activity of the particular enzyme, the concentration of reactants and/or products in the recycle stream, whether the enzyme is soluble or immobilized, the enzyme concentration if soluble, desired purity of the product, the particular means of separation, and so forth. Under many process conditions the location of the recycle point may be varied broadly without substantial impact. For instance, where the enzyme is immobilized, a portion of the glucose-depleted stream may be recycled to join the feed prior to entering the hydrolysis zone. But in any event it should be clear that the determination of a suitable entry point will depend upon the specific parameters utilized in any particular process with its determination well within the capability of the skilled worker.

As heretofore set forth, the enzymes which are utilized in the process of this invention most often comprise amyloglucosidase or betaamylase which may be composited on a solid support. By utilizing enzymes which are immobilized on a support, it is possible to stabilize the enzyme in a relative manner and therefore to permit effective use of enzyme which otherwise might be lost in the reaction medium. Such immobilized or insolubilized enzymes may be employed in various reactor systems such as in packed columns, stirring tank reactors, etc., depending upon the nature of the substrate which is utilized therein. By permitting the reuse of the enzymes which are in a relatively stable condition and thus may be utilized for a relatively lengthy period of time, it is possible to operate the process in a commercially attractive and economical manner.

The particular enzyme may be immobilized on a solid support in any manner known in the art. One such method of immobilizing the enzyme which may be used as an illustration of a method for immobilizing the enzyme is taught in U.S. Patent 4,141,857.

As hereinbefore set forth, it is also contemplated within the scope of this invention that ultra-filtration membranes may be used to recover glucose or maltose from a partially hydrolyzed reaction mixture resulting from the treatment of a liquid starch feedstock with an enzyme. Any membrane which possesses an appropriate molecular weight as well as pore sizes capable of yielding the desired high glucose or maltose content as the permeate while retaining the rejected material which contains unhydrolyzed oligosaccharides is suitable. The ultra-filtration membrane will possess a molecular weight cut-off in the range of from about 100 to about 5000 and will also possess pore sizes in the range of from about 5 to about 125 Angstroms. Some specific examples of these membranes which may be utilized will include cellulose acetates such as cellulose diacetate, cellulose triacetate or mixtures thereof, a membrane resulting from polyethyleneimine crosslinked with a dialdehyde, a diacidchloride, or a diisocyanate, polyacrolein, chitosan cross-linked with a dialdehyde such as glutaraldehyde, polystyrenesulfonates, etc. It is to be understood that these ultra-filtration membranes are only representative of the class of membranes which may be employed and that the present invention is not necessarily limited thereto.

The two-step process of the present invention may be effected in any suitable manner and may comprise either a batch or continuous type operation. For example, when a batch type operation is employed, a quantity of the feedstock comprising a liquid starch which has been previously treated with alpha-amylase to increase the dextrose equivalent is contacted with an immobilized amyloglucosidase, in the event that a syrup high in glucose content is desired, for a predetermined period of time while employing reaction conditions which include a temperature of from about 45° to about 70°C and a pressure which may range from about 1 to about 1000 psi. In addition, the residence time during which the feedstock is in contact with the immobilized enzyme is correlated with the liquid hourly space velocity at which the feed is introduced so as to produce a conversion of the liquid starch to glucose within the desired range. After passage over the immobilized enzyme, the effluent is recovered and the partially hydrolyzed starch is then subjected to an ultrafiltration step. In this step, the reaction mixture is passed through an ultrafiltration membrane of the type hereinbefore set forther whereby the permeate which possesses a high glucose or maltose content is recovered as the permeate upon separation from the retentate. The latter may then also be recycled, a portion of the recycle stream being admixed with the effulent from the enzyme treatment while another portion of the retentate is admixed with the feede stream to the immobilized enzyme treatment zone. It is also contemplated that a third portion, if so desired, may be recycled back to the zone in which the feedstock is pretreated with alpha-amylase.

It is also contemplated within the scope of this invention that the process may be effected in a continuous manner of operation. When this type of operation is employed, the feedstock comprising the treated liquified starch is continuously charged to a reaction vessel such as a column which contains the desired immobilized enzyme, said column being maintained at the proper operating conditions of temperature and pressure. After passage over the enzyme at a predetermined liquid hourly space velocity which is sufficiently high so as to only partially hydrolyze the feedstock, with a concomitant low or nonexistent production of reversion products such as isomaltose, the effluent is continuously withdrawn and charged to an ultrafiltration apparatus which contains a membrane of the type hereinbefore set forth. After passage through this apparatus, which is also maintained at the proper operating conditions of temperature and pressure, the permeate comprising syrup which has a high glucose or maltose content, i.e., above 90%, is recovered. The retentate material which contains unhydrolyzed oligosaccharides such as those which have a DP rating of $DP_7$, $DP_8$, $DP_{9+}$ (the designation DP being the degree of polymerization) is also recovered and a portion thereof recycled back to the column containing the immobilized enzyme for further use as a portion of the feedstock, another portion admixed with the effluent from the immobilized enzyme treatment and, if so desired, a portion to the alpha-amylase step for pretreatment.

5

Each of the heretofore mentioned membrane separations may be performed using solid adsorbents as well. One such adsorbent is disclosed in U.K. Patent No. 1,585,369, the teachings of which are incorporated herein by reference, and comprises X or Y zeolites containing one or more selected cations at the exchangeable cationic sites. Potassium -X zeolite is a particularly preferred adsorbent exhibiting selectivity for glucose with respect to disaccharides and higher oligosaccharides and the polysaccharides.

The following eleven examples are given for the purpose of illustrating the present invention. However, it is to be understood that these examples are given merely for purposes of illustration and that the present process is not necessarily limited thereto.

Example I

To illustrate the process of the present invention, a starch feedstock was treated with alpha-amylase to adjust the dextrose equivalent to 15 DE. In this case, the enzyme was immobilized and comprised amylglucosidase composited on a solid alumina support. The treated starch feedstock was passed through a column of 40 cc of the immobilized enzyme, said starch feedstock containing 0.1% benzoate and 50 ppm of sodium omadine at a pH of 4.2. The starch was treated at a temperature of 45°C, and a pressure greater than atmospheric at a liquid hourly space velocity of 3.21. The effluent from this column was analyzed by means of liquid chromatography. The analysis showed the following area % in which DP is the degree of polymerization (for example, $DP_7$ = seven (7) monomers of glucose in the oligosaccharide):

| $DP_{9+}$ | $DP_8$ | $DP_7$ | $DP_6$ | $DP_5$ | $DP_4$ | $DP_3$ | $DP_2$ | Glucose |
|---|---|---|---|---|---|---|---|---|
| 19.1 | 0.2 | 0.1 | — | — | — | 0.3 | 3.1 | 77.2 |

The effluent in an amount of 200 cc which was recovered from this column was then passed through a cellulose acetate membrane, 130.7 cc out of the original 200 cc being obtained and constituting the permeate. Analysis of the permeate after passage through the membrane at a temperature of 22°C and a pressure of 90 psi showed that said permeate contained 94.2% glucose, 3.6% maltose and only 1.7% of the oligosaccharides having a DP of 9+.

The retentate which is recovered from the treatment with the cellulose acetate membranes may then be recycled and utilized as a portion of the feedstock which is charged to the zone containing the immobilized enzyme comprising amyloglucosidase composited on the treated alumina support.

Example II

In a manner similar to that hereinbefore set forth in Example I, a treated liquified starch feedstock was again passed through an immobilized amyloglucosidase column under conditions similar to those in Example I. The effluent from this column, which contained 77.2% glucose as well as minor amounts of maltose, $DP_3$, $DP_7$, $DP_8$ and a major amount of $DP_{9+}$ oligosaccharides, was passed through a membrane comprising plystyrenesulfonate and sold under the trade name Amicon UM2. The volume of the effluent which passed through the membrane was 122.8 cc. Analysis of the permeate by means of liquid chromatography showed 97.6% glucose, 2.3% maltose, and only 0.1% of $DP_{9+}$ oligosaccharides.

When the above experiment was repeated using a membrane comprising cellulose acetate sold under the trade name Nuclepore and the effluent from the immobilized amyloglucosidase column was passed over this membrane at a temperature of 22°C and a pressure of 90 psi, the permeate was found to contain 97.5% glucose, 2.3% maltose, and 0.2% $DP_{9+}$ oligosaccharides.

Likewise, the retentate which may be recovered from the treatment of the effluent with the cellulose acetate membrane may be recovered and recycled, a portion of the retentate being admixed with the effluent from the immobilized enzyme treatment while the other portion is recycled to form a portion of the feedstock which is charged to the immobilized enzyme treatment zone.

Example III

To illustrate the ability of the process of the present invention to recover a product containing a high maltose concentration, a liquified starch feedstock which had been pretreated with alpha-amylase to adjust the dextrose equivalent to the desired level was passed through a column containing 100 cc of beta-amylase immobilized on a solid matrix similar to that set forth in Example I above. The feestock which had a pH of 5.0 contained 0.1 mole of acetate and 0.2 mole of benzoate which acted as a buffer. The feedstock was passed over the enzyme at a temperature of 55°C, a pressure greater than atmospheric, at a liquid hourly space velocity of 5. The effluent which was recovered from this column was subjected to liquid chromatography analysis with the following result:

HPLC ANALYSIS (AREA %)

| $DP_{9+}$ | $DP_8$ | $DP_7$ | $DP_6$ | $DP_5$ | $DP_4$ | $DP_3$ | $DP_2$ | Glucose |
|---|---|---|---|---|---|---|---|---|
| 36.9 | 0.9 | 0.3 | 0.3 | 0.1 | 0.5 | 11.1 | 50.2 | .5 |

The effluent from this column in an amount of 100 cc was passed through a cellulose acetate membrane while maintaining the membrane apparatus at a temperature of 22°C and a pressure of 90 psi.

6

Analysis of the permeate by means of liquid chromatography showed the presence of 79.0% maltose, 16.3% of a $DP_3$ product and 2.4% of a $DP_{9+}$ oligosaccharide.

When the above experiment was repeated using an ultrafiltration membrane sold under the trade name of Amicon UM2, the permeate was found to contain 90.1% maltose, 9.7% of a $DP_3$ oligosaccharide and only 0.2% of a $DP_{9+}$ oligosaccharide.

In a similar manner the retentate which is recovered from the treatment with the ultrafiltration membrane of the above paragraph may be recycled, a portion being admixed with the effluent withdrawn from the treatment with the immobilized beta-amylase while another portion may be admixed with the feedstock comprising the liquified starch prior to passage over the immobilized enzyme.

Example IV

In this example, a liquified starch feedstock which may be pretreated with alpha-amylase to adjust the dextrose equivalent of said starch to a predetermined level may be passed through an immobilized amyloglucosidase column at a temperature of 45°C and a pressure greater than atmospheric. The effluent from the column may be withdrawn after having been in contact with the immobilized enzyme for a predetermined period of time sufficient to permit a conversion to glucose of about 75% and may then be passed through an ultrafiltration membrane comprising polyacrolein, said passage over the membrane being effected at conditions which will include ambient temperature and a pressure of about 25 psi. The permeate may be recovered while the retentate may be recycled to be admixed with the effluent prior to passage over the aforesaid membrane.

In a similar manner, a liquified starch feedstock which contains a dextrose equivalent of about 15 may also be passed over an immobilized enzyme comprising beta-amylase composited on a treated alumina support, the reaction conditions including a temperature of about 50°C and a pressure greater than atmospheric. After passage over the enzyme at a predetermined liquid hourly space velocity and for a residence time sufficient to permit a conversion of about 75% of the starch to glucose, the effluent may be continuously withdrawn and passed over the membrane comprising polyethyleneimine dialdehyde, said passage over the membrane being effected at a temperature of about 25°C and a pressure of about 100 psi. The permeate which may contain over 90% glucose may be recovered while the retentate may be recycled, one portion of which may be admixed with the effluent, withdrawn from the enzyme treatment zone, a second portion may be recycled to be admixed with the liquified starch feedstock entering the immobilized enzyme zone, while a third portion may be recycled to the pretreatment zone in which the starch feedstock is contacted with alpha-amylase, the alpha-amylase acting to raise the dextrose equivalent of the starch to a predetermined level.

The effluent which is continuously withdrawn from the treatment with the immobilized enzyme may also be passed over an ultrafiltration membrane comprising chitosan dialdehyde at reaction conditions which include a temperature of about 22°C and a pressure of about 100 psi, the permeate comprising a major portion of glucose being recovered while the retentate may be recycled to be admixed with the effluent which is dispersed from the immobilized enzyme treatment zone.

The seven examples which follow are merely illustrative of the invention and are not intended to limit or restrict it in any way.

Thinned starch used as the feedstock was Maltrin-150, a typical analysis for which showed about 1% glucose, 3% $DP_2$, 6% $DP_3$, 4% $DP_4$, 4% $DP_5$, 9% $DP_6$, 16% $DP_7$, and 58% $DP_8$ and higher.

Glucoamylase was assayed as follows. To 4 ml of a starch solution, 30% dry solids, was added 25 microliters of the enzyme solution, and the mixture was incubated 30 minutes at 60°C. Hydrolysis was quenched by the addition of 1 ml 0.2N NaOH, and the mixture was cooled. The amount of glucose formed was determined using a glucose analyzer. The number of grams of glucose produced per hour is the AG activity expressed in Miles units.

Example V

A solution of AG (77 units/liter) at pH 4.2 in glucose solutions containing varying levels of dry solids was maintained at 60°C. Aliquots were withdrawn periodically and assayed for AG activity. The enzyme half-life was found to be 4.8, 12.3, and 21.9 days at dry solids levels of 30%, 44% and 55%, respectively. Thus, enzyme stability as measured by half-life at 60°C is increased over 4.5-fold in going from 30% to 55% dry solids.

Example VI

Feedstocks of Maltrin-150 of different dry solids (DS) level at pH 4.2 containing AG at 77 units per liter were hydrolyzed at 60°C. Glucose concentration as determined by high pressure liquid chromatography was monitored with time, the results being summarized in the accompanying table.

7

**0 176 621**

TABLE 1
Glucose Concentration with Hydrolysis Time

| Time (hours) | Glucose conc., grams/liter | | |
|---|---|---|---|
| | 30% DS | 44% DS | 55% DS |
| 5 | 260 | 290 | 305 |
| 10 | 320 | 405 | 425 |
| 20 | 330 | 500 | 540 |
| 40 | 340 | 525 | 600 |
| 80 | 340 | 525 | 600 |

The results clearly show that glucose production rates increases with increasing dry solids level of feedstock.

Example VII

A feedstock of Maltrin-150, 30% dry solids, at pH 4.2 and containing AG at 77 units per liter was hydrolyzed at 60°C. Product was analyzed for glucose, total disaccharides (DP$_2$), and isomaltose. Results using two different lots of AG are summarized in the table below.

TABLE 2
Glucose, Disaccharide, and Isomaltose Production

| Run 1 | Time (hours) | glucose | Weight % | |
|---|---|---|---|---|
| | | | disaccharides | isomaltose |
| | 2 | 28 | 8.5 | 0 |
| | 4 | 58 | 9.0 | 0.6 |
| | 6 | 72 | 4.5 | 0.9 |
| | 8 | 88 | 4.0 | 0.9 |
| | 10 | 90 | 4.5 | 1.3 |
| | 12 | 92 | 4.8 | 1.3 |
| | 20 | 94 | 5.8 | 2.2 |
| | 30 | 94 | 7.0 | 3.4 |
| Run 2 | 1.8 | 45.1 | 17.3 | 0 |
| | 4.7 | 69.0 | 10.8 | 0 |
| | 5.6 | 74.4 | 7.5 | 0 |
| | 7.5 | 80.9 | 4.5 | 0 |
| | 13.0 | 91.1 | 3.2 | 0.0 |
| | 20.0 | 93.5 | 3.4 | 0.8 |
| | 48.0 | 94.8 | 4.1 | 1.6 |

Thus, relative to the time needed to attain a 94% glucose product attainment of a 90% glucose product takes about half the time, and attainment of a 92% glucose product takes about 60% of the time.

These results also clearly show the accumulation of isomaltose during the latter stages of conversion.

8

## Example VIII

This example compares results from a once-through reactor, i.e., a conventional operation in which thinned starch is hydrolyzed to about 94% or greater glucose, with a closed-loop system where reactor effluent is sent to a membrane, a glucose-enriched product stream is drawn off, and the glucose-depleted stream is recycled to the beginning of the reactor. All hydrolyses were performed at 60°C, pH 4.5—5.5. Run A is the conventional, once-through reactor; both Runs B and C are closed-loop systems, with B using a cellulose acetate membrane having a 10,000 molecular weight cutoff operating at 60°C, 150 psig, and C using a polyelectrolyte membrane with a 500 molecular weight cutoff operating at 60°C, 300 psig, both supplied by Amicon Co. under their designations YM10 and UM05, respectively. Results are listed in Table 3, where $DP_1$ represents total monosaccharides, nearly all of which is glucose, $DP_2$ represents disaccharides, $DP_{4+}$ are polysaccharides or four or more units, and DS is dry solids. For Runs B and C reactor effluent, product and recycle stream analyses are equilibrium values.

TABLE 3
Comparison of Once-Through (Single Pass)
Reactor with Closed-Loop, Recycle Reactor

|  | Run A | Run B | Run C |
|---|---|---|---|
| Enzyme (AG) dosage, units/liter | 77 | 34 | 58 |
| Residence time, hours | 48 | 14 | 20 |
| Feedstock Composition (%) |  |  |  |
| DS | 30.0 | 26.1 | 24.7 |
| $DP_1$ | 1.0 | 1.2 | 1.6 |
| $DP_2$ | 4.5 | 4.3 | 3.9 |
| $DP_{4+}$ | 87.5 | 87.8 | 88.3 |
| Reactor Effluent |  |  |  |
| DS | 33.0 | 27.0 | 30.4 |
| $DP_1$ | 95.7 | 90.5 | 83.4 |
| $DP_2$ | 4.1 | 2.9 | 4.4 |
| $DP_{4+}$ | 0.2 | 6.3 | 11.6 |
| Product Stream |  |  |  |
| DS |  | 26.1 | 24.7 |
| $DP_1$ |  | 93.6 | 98.0 |
| $DP_2$ |  | 3.1 | 2.0 |
| $DP_{4+}$ |  | 2.9 | 0.0 |
| Recycle Stream |  |  |  |
| DS |  | 27.4 | 34.2 |
| $DP_1$ |  | 89.9 | 82.5 |
| $DP_2$ |  | 3.5 | 3.8 |
| $DP_{4+}$ |  | 6.3 | 13.1 |

## Example IX

A kinetic model was developed from experimental data which not only closely reproduced existing experimental data but also had reliably high predictive ability. Among the variables (experimental parameters) accommodated by the model were feedstock (thinned starch) composition, enzyme dosage, reactor residence time, membrane type and operating conditions. Output included glucose and isomaltose concentration in the product stream. The feedstock of thinned starch had 30.6 weight percent dry solids with 1.2% $DP_1$, 4.8% $DP_2$, and 87% $DP_{4+}$. Run A represents a once-through reactor, and Runs B and C represent a closed-loop recycle reactor using the polyelectrolyte membrane described in the prior example with the glucose-depleted stream recycled to the top of the reactor. Reactor effluent and product compositions are equilibrium values.

**0 176 621**

TABLE 4
Isomaltose Accumulation

|  | Run A | Run B | Run C |
|---|---|---|---|
| AG concentration, units/liter | 77 | 9 | 18 |
| Residence time, hours | 48 | 10 | 10 |
| Reactor effluent: % glucose | 95.7 | 74 | 87.3 |
| % isomaltose | 1.66 | 0.7 | 1.85 |
| Product: % glucose |  | 95.5 | 97.2 |
| % isomaltose |  | 0.34 | 0.73 |

These data clearly show that the closed-loop, recycle process produces glucose at as high concentration as that from a conventional, once-through reactor at substantially lower enzyme dosage, with one-fourth the isomaltose content. In fact, Run C shows that one can increase glucose in the product to over 97% (using one-fourth the enzyme concentration) and still have less than half the isomaltose content as that from the conventional process.

Example X

In this example the aforementioned kinetic model was used to determine the effect of recycle location on glucose and isomaltose concentration in the steady state product and reactor effluent streams. Data are summarized in Table 5, where "reactor volume" refers to the percent reactor volume prior to the recycle location; 0 represents the top, 100 represents the bottom of the reactor. Thinned starch feedstock had the composition of that in the foregoing example; reactor time was 10 hours and the polyelectrolyte membrane of Example 4 was used under the stated conditions.

TABLE 5
Effect of Recycle Point on Product Composition

|  | Reactor Volume | | | | | |
|---|---|---|---|---|---|---|
|  | 0 | 30 | 50 | 70 | 90 | 95 |
| AG concentration: 18 u/l |  |  |  |  |  |  |
| Reactor effluent |  |  |  |  |  |  |
| glucose | 87.4 | 88.0 | 87.0 | 85.0 | 73.0 | 57.0 |
| isomaltose | 1.85 | 1.8 | 1.7 | 1.3 | 0.6 | 0.25 |
| Product |  |  |  |  |  |  |
| glucose | 97.2 | 97.2 | 97.2 | 96.9 | 96.9 | 90.7 |
| isomaltose | 0.73 | 0.71 | 0.66 | 0.55 | 0.27 | 0.13 |
| AG concentration: 77 u/l |  |  |  |  |  |  |
| Reactor effluent |  |  |  |  |  |  |
| glucose | 91.5 | 91.5 | 91.5 | 91.5 | 92.3 | 91.0 |
| isomaltose | 4.75 | 4.8 | 4.7 | 4.4 | 3.35 | 2.5 |
| Product |  |  |  |  |  |  |
| glucose | 96.9 | 96.9 | 96.9 | 97.0 | 97.3 | 97.4 |
| isomaltose | 1.73 | 1.74 | 1.70 | 1.59 | 1.22 | 0.93 |

The results in the table reflect the fact that at high AG concentration the product composition is rather insensitive to recycle point throughout, and at lower enzyme concentration no substantial change in product composition is seen when the recycle point is between 0—90% of reactor volume. The overall conclusion is that recycle location will be a process choice depending upon other process parameters such as enzyme concentration, residence time, separation means used, product composition desired, etc.

10

Example XI

These data generated by the kinetic model show the advantages, absent in a once-through reactor, accruing from a feedstock with high dry solids used in a closed loop, recycle process. Feedstock had the composition of Example 5 with only the dry solids (weight percent) varying. The recycle reactor utilized the polyelectrolyte membrane of Example 4 under the conditions stated therein.

TABLE 6
Effect of Dry Solids Variation

|  | Once-through reactor | | Closed loop recycle reactor |
|---|---|---|---|
| % Dry solids | 30 | 40 | 40 |
| Residence time, hours | 48 | 71 | 10 |
| AG concentration, units/liter | 77 | 77 | 18 |
| Product, wt.%: glucose | 95.7 | 93.6 | 95.6 |
| isomaltose | 1.7 | 2.2 | 0.7 |

In a once-through reactor residence time is unacceptably long and isomaltose concentration is unacceptably high using 40% dry solids feedstock, whereas in the closed loop recycle reactor configuration residence time is reduced 7-fold, isomaltose concentration 3-fold, and enzyme concentration 4-fold.

**Claims**

1. A process for selectively obtaining a sugar which is either glucose or maltose from thinned starch comprising hydrolyzing a feedstock of thinned starch under the action of a glucose- or maltose-producing enzyme, separating the effluent stream into a sugar-enriched product stream and a sugar-depleted stream, recovering the sugar-enriched product stream, and recycling the sugar-depleted stream to the hydrolysis step, characterized in that the feedstock is hydrolyzed to an effluent stream containing from 50% to 92% of said sugar.

2. The process of claim 1 where the feedstock contains from about 30% to about 45% dry solids.

3. The process of claim 1 or 2 where the effluent stream contains from about 60% to about 85%, preferably from about 70% to about 80% sugar.

4. The process of one of the claims 1 to 3 where the hydrolysis step is catalyzed by a soluble enzyme.

5. The process of one of the claims 1 to 4 where the hydrolysis step is catalyzed by an immobilized enzyme.

6. The process of one of the claims 1 to 5 where the enzyme is amyloglucosidase and the sugar is glucose.

7. The process of one of the claims 1 to 6 where the enzyme is beta-amylase and the sugar is maltose.

8. The process of one of the claims 1 to 7 where the sugar-enriched stream contains at least about 90% sugar.

9. The process of one of the claims 1 to 8 where a membrane-based separation step is utilized.

10. The process of one of the claims 1 to 9 where a solid adsorbent-based separation step is utilized.

**Patentansprüche**

1. Verfahren zur selektiven Gewinnung eines Zuckers, der entweder Glukose oder Maltose ist, aus verdünnter Stärke durch Hydrolysieren einer Beschickung von verdünnter Stärke unter der Wirkung eines Glukose oder Maltose erzeugenden Enzyms, Trennung des Auslaufstromes in einen an Zucker angereicherten Produktstrom und einen an Zucker verarmten Strom, Gewinnung des an Zucker angereicherten Produktstromes und Rückführung des an Zucker verarmten Stromes zu der Hydrolysestufe, dadurch gekennzeichnet, daß die Beschickung zu einem Auslaufstrom hydrolysiert wird, der 50% bis 92% des Zuckers enthält.

2. Verfahren nach Anspruch 1, bei dem die Beschickung etwa 30% bis etwa 45% Trockenfeststoffe enthält.

3. Verfahren nach Anspruch 1 oder 2, bei dem der Auslaufstrom etwa 60% bis etwa 85%, vorzugsweise etwa 70% bis etwa 80% Zucker enthält.

4. Verfahren nach einem der Ansprüche 1 bis 3, bei dem die Hydrolysestufe durch ein lösliches Enzym katalysiert wird.

5. Verfahren nach einem der Ansprüche 1 bis 4, bei dem die Hydrolysestufe durch ein immobilisiertes Enzym katalysiert wird.

6. Verfahren nach einem der Ansprüche 1 bis 5, bei dem das Enzym Amyloglukosidase und der Zucker Glukose ist.

7. Verfahren nach einem der Ansprüche 1 bis 6, bei dem das Enzym beta-Amylase und der Zucker Maltose ist.

8. Verfahren nach einem der Ansprüche 1 bis 7, bei dem der an Zucker angereicherte Strom wenigstens etwa 90% Zucker enthält.

9. Verfahren nach einem der Ansprüche 1 bis 8, bei dem eine Trennstufe auf membranbasis benutzt wird.

10. Verfahren nach einem der Ansprüche 1 bis 9, bei dem eine Trennstufe auf Adsorbensbasis benutzt wird.

## Revendications

1. Procédé pour obtenir sélectivement un sucre qui est soit du glucose, soit du maltose, à partir d'amidon fluide, consistant à hydrolyser une charge d'amidon fluide sous l'action d'une enzyme produisant du glucose ou du maltose, à séparer le courant d'effluent en un courant de produit enrichi en sucre et en un courant appauvri en sucre, à récupérer le courant de produit enrichi en sucre et à recycler le courant appauvri en sucre vers l'étape d'hydrolyse, caractérisé en ce que la charge est hydrolysée pour donner un courant d'effluent contenant de 50 à 92% dudit sucre.

2. Procédé selon la revendication 1, dans lequel la charge a un extrait sec d'environ 30 à environ 45%.

3. Procédé selon la revendication 1 ou 2, dans lequel le courant d'effluent contient d'environ 60 à environ 85%, de préférence d'environ 70 à environ 80% en sucre.

4. Procédé selon l'une des revendications 1 à 3, dans lequel l'étape d'hydrolyse est catalysée par une enzyme soluble.

5. Procédé selon l'une des revendications 1 à 4, dans lequel l'étape d'hydrolyse est catalysée par une enzyme immobilisée.

6. Procédé selon l'une des revendications 1 à 5, dans lequel l'enzyme est l'amyloglucosidase et le sucre est le glucose.

7. Procédé selon l'une des revendications 1 à 6, dans lequel l'enzyme est la bêta-amylase et le sucre est le maltose.

8. Procédé selon l'une des revendications 1 à 7, dans lequel le courant enrichi en sucre contient au moins environ 90% de sucre.

9. Procédé selon l'une des revendications 1 à 8, dans lequel on utilise une étape de séparation fondée sur une membrane.

10. Procédé selon l'une des revendications 1 à 9, dans lequel on utilise une étape de séparation fondée sur un adsorbant solide.

## FIG. I

Conventional Process

Thinned Starch

Saccharification

Evaporation

Glucose ≥ 94%

Isomerization 35-50% DS

## FIG. 2

Improved Process

Thinned Starch

Saccharification

(Glucose Depleted)

Glucose 50-92%

Separation

Glucose ≥ 94%

Evaporation

Isomerization 35-50% DS